# FASCICULE DE BREVET EUROPEEN

(11) **EP 0 813 888 B1**
(45) Date de publication et mention de la délivrance du brevet: **18.02.2004**
(21) Numéro de dépôt: 97401388.0
(22) Date de dépôt: 18.06.1997
(51) Int. Cl.: A61N 1/365, A61N 1/39

(54) **Dispositif médical actif du type défibrillateur-cardioverteur implantable à discrimination perfectionnée des tachycardies**
Aktive medizinische Vorrichtung von der Art eines Defibrillator-Kardiovertierer mit verbesserter Unterscheidung von Tachykardien
Defibrillator/cardioverter type active medical device with improved discrimination of tachycardia

(30) Priorité: 18.06.1996 FR 9607533
(43) Date de publication de la demande: 29.12.1997
(73) Titulaire: ELA MEDICAL (Société anonyme), 92541 Montrouge (FR)
(72) Inventeur: Nitzsché, Rémi, 78950 Gambais (FR); Bonnet, Jean-Luc, 92120 Montrouge (FR); Iscolo, Nicolas, 78120 Saint Cyr L'Ecole (FR); Limousin, Marcel, 92120 Montrouge (FR)
(74) Mandataire: Dupuis-Latour, Dominique

(56) Documents cités:
- EP-A- 0 469 817
- EP-A- 0 540 141
- EP-A- 0 550 344
- WO-A-94/16768
- US-A- 4 860 749
- US-A- 5 193 535
- US-A- 5 243 980

## Description

La présente invention concerne les dispositifs médicaux implantables actifs (au sens de la directive 90/385/CEE du 20 juin 1990 du Conseil des communautés européennes), et plus particulièrement la famille des appareils chargés de délivrer au coeur des impulsions électriques de haute énergie (c'est-à-dire dépassant notablement l'énergie fournie pour la simple stimulation) en vue de mettre fin à une tachyarythmie. Ces modes de thérapie incluent également un mode de stimulation programmée à haute fréquence ou "ATP" (AntiTachycardic Pacing).

Ces dispositifs sont communément appelés "défibrillateurs implantables" ou "appareils de cardioversion" (étant entendu que l'invention couvre aussi bien les défibrillateurs/cardioverteurs/stimulateurs implantables que les défibrillateurs/stimulateurs implantables).

Ces dispositifs comprennent un générateur d'impulsions chargé de surveiller l'activité cardiaque et de générer des impulsions de stimulation ou de haute énergie quand le coeur présente une arythmie ventriculaire susceptible d'être traitée (quand cette énergie est comprise entre 0,1 et 10 J environ, on désigne cette thérapie sous le nom de "cardioversion" et le choc électrique est appelé "choc de cardioversion". Quand cette énergie est supérieure à 10 J environ, le choc électrique est alors appelé "choc de défibrillation").

Ce choc doit être délivré lorsque l'on détecte une tachycardie ventriculaire (TV), mais à condition qu'il s'agisse bien d'une véritable TV, et non d'une tachycardie supra-ventriculaire (TSV) ; en effet, dans ce dernier cas, la tachycardie est d'origine auriculaire et le choc qui serait délivré serait sans effet puisque l'électrode de défibrillation ou, le cas échéant, de stimulation, ne se trouve pas dans cette région.

La tachyarythmie correspond à un rythme cardiaque rapide anormal et recouvre la fibrillation ventriculaire (FV), la tachycardie ventriculaire (TV), la tachycardie sinusale (TS) et la tachycardie supra-ventriculaire (TSV). La tachycardie supra-ventriculaire recouvre la tachycardie auriculaire, le *flutter* auriculaire et la fibrillation auriculaire.

Le diagnostic des tachycardies est opéré, de manière en elle-même connue (voir notamment le EP-A-0 626182 au nom de ELA Médical), à partir de critères tels que la fréquence ventriculaire, la stabilité des intervalles ventriculaires (intervalles RR), l'analyse de l'association auriculo-ventriculaire et le mode de démarrage des tachycardies (présence d'accélération et cavité d'origine, ventriculaire ou auriculaire).

L'algorithme de diagnostic permettant la détection des tachycardies et leur classification (c'est-à-dire la discrimination entre TSV et TV) est conçu de manière à procurer une sensibilité maximale -pour détecter toutes les tachycardies ventriculaires, évitant ainsi les faux diagnostics négatifs - tout en préservant la spécificité de la discrimination - c'est-à-dire en discriminant bien les TSV des TV, évitant ainsi les faux diagnostics positifs (indication d'une TV alors qu'il s'agit d'une TSV).

De plus, cet algorithme permet de détecter les fibrillations auriculaires (FA), c'est-à-dire les fréquences anormalement élevées du rythme auriculaire et de bien distinguer une FA isolée (ne devant pas entraîner la délivrance d'une thérapie ventriculaire) d'une TV (devant entraîner la délivrance rapide d'une thérapie ventriculaire).

On observe cependant dans certains cas des défaillances de l'algorithme de classification, notamment en présence d'une FA conduite et installée présentant des intervalles RR réguliers pendant une durée suffisante pour conduire à un faux diagnostic de TV (rythme régulier, dissocié entre oreillettes et ventricules).

Si l'on diminue la sensibilité de l'algorithme pour éviter de tels faux diagnostics, on risque d'aboutir soit à une sous-détection des TV, soit à un allongement de la durée d'analyse de l'algorithme, avec pour inconvénients, dans le premier cas, le risque de non-détection de TV réelles et, dans le second cas, un allongement du délai entre la détection de la TV et l'application de la thérapie, allongement du délai qui est fortement préjudiciable au patient.

L'un des buts de l'invention est de résoudre cette difficulté, en proposant de perfectionner les dispositifs existants afin d'éliminer tous risque de faux diagnostic de TV en présence de FA conduite et installée, c'est-à-dire d'accroître la spécificité de l'analyse des tachycardies sans pour autant compromettre la sensibilité de la détection de celles-ci, lorsqu'elles démarrent sur un rythme sinusal.

Le dispositif médical de l'invention est un défibrillateur ou cardio-verteur de type en lui-même connu, c'est-à-dire qu'il comprend : des moyens de délivrance d'impulsions à haute énergie ; des moyens de recueil de l'activité ventriculaire et auriculaire ; des moyens pour suspecter et confirmer la présence d'épisodes de tachycardie dans l'activité ainsi recueillie ; et des moyens classificateurs, propres à discriminer les tachycardies ainsi détectées entre tachycardies ventriculaires et tachycardies supra-ventriculaires en fonction de critères prédéterminés et d'un paramétrage donné de ces critères, et à autoriser le déclenchement des moyens de délivrance de la thérapie en présence de tachycardies ventriculaires et inhiber ces mêmes moyens en présence de tachycardies supra-ventriculaires.

Selon l'invention, les moyens classificateurs comprennent des moyens propres à ajouter temporairement un ou plusieurs critères et/ou à modifier temporairement le paramétrage d'au moins l'un des critères existants en cas de tachycardie supra-ventriculaire.

Les critères ajoutés peuvent notamment comprendre un critère supplémentaire de recherche d'intervalles RR courts et/ou longs significativement éloignés d'une zone de stabilité donnée ; les zones d'éloignement significatif sont par exemple séparées des bornes de la zone de stabilité par un intervalle de largeur prédéterminée, identique en-deçà et au-delà de la zone de stabilité.

Les critères ajoutés peuvent également comprendre un critère supplémentaire de comptabilisation et de comparaison du nombre d'événements auriculaires par rapport à celui des événements ventriculaires pendant une durée donnée.

Le paramétrage modifiable peut comprendre : un paramètre de majorité dans lequel on évalue, sur un nombre de cycles donné, la présence d'un pourcentage, supérieur ou égal à un seuil donné, d'intervalles RR situés hors d'une zone de stabilité donnée ; un paramètre de durée de persistance des tachycardies ; et un paramètre de largeur d'une zone de stabilité des intervalles RR.

Les critères et/ou les paramètres des moyens classificateurs peuvent être restaurés lorsque des épisodes de tachycardie supra-ventriculaire ne sont plus confirmés dans l'activité recueillie.

On peut également prévoir que, si un ou plusieurs critères sont ajoutés temporairement et/ou le paramétrage modifié temporairement un nombre déterminé de fois au cours d'une période donnée, cet ajout et/ou cette modification sont alors appliqués de façon permanente.

Enfin, le dispositif peut comprendre en outre des moyens pour délivrer une thérapie auriculaire en réponse à la détection d'une tachycardie supra-ventriculaire.

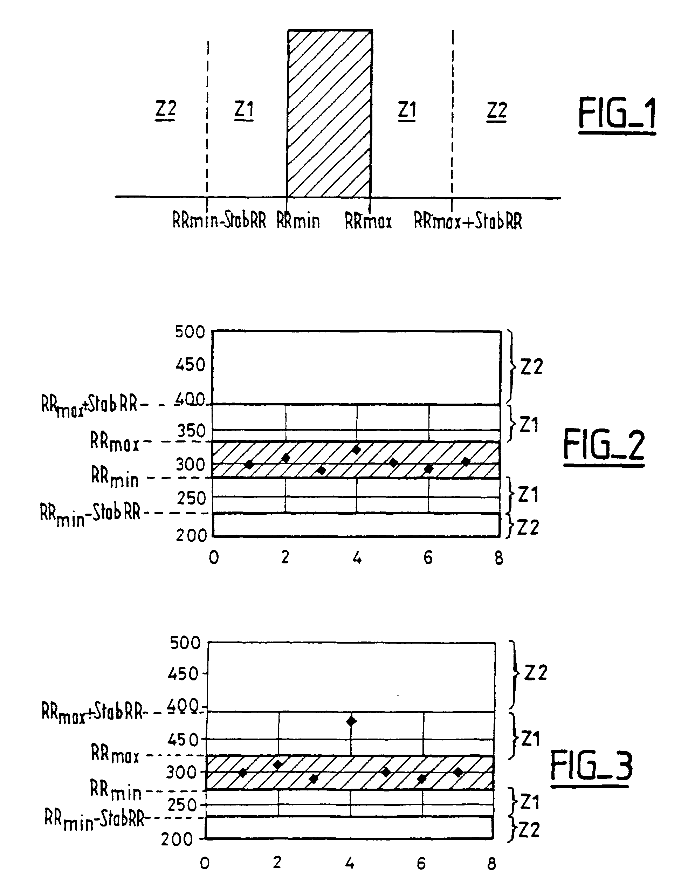

D'autres caractéristiques de l'invention apparaîtront à la lecture de la description détaillée ci-dessous, en référence aux figures 1 à 3 annexées qui illustrent le critère supplémentaire de recherche de cycles longs ou courts significativement éloignés de la zone de stabilité.

L'invention consiste, comme on l'a exposé plus haut, à détecter une activité électrique qui laisse suspecter la présence d'une FA permanente ou chronique de rythme régulier ("FA installée" ou "FA conduite") et, si tel est le cas, à modifier temporairement les paramètres de classification des tachycardies et/ou les critères de classification de ces tachycardies, ceci afin d'augmenter la spécificité de discrimination du système sans pour autant diminuer de façon significative sa sensibilité aux TV.

Parmi les paramètres qui sont actuellement utilisés, et dont on peut se contenter de modifier la programmation, on trouve essentiellement :
- le paramètre de majorité (ou "critère majoritaire"), selon lequel on effectue une analyse statistique d'un histogramme des intervalles RR mémorisés au cours d'un nombre donné de cycles (paramétrage nominal : 8 cycles) et l'on recherche si l'on est en présence ou non d'un pourcentage d'intervalles RR situés en dehors du pic central de stabilité qui est supérieur à une valeur donnée (paramétrage nominal : 75 %) ; si tel est le cas, on considère que le rythme est régulier et qu'il n'y a pas tachycardie,
- la durée de persistance de l'épisode de tachycardie (paramétrage nominal : 8 cycles),
- la largeur du pic de stabilité utilisée pour la détection de régularité du rythme (paramétrage nominal : 63 ms).

Ces paramètres sont par exemple mis en oeuvre dans l'algorithme de détection et de classification des tachycardies d'un défibrillateur tel que le modèle *Defender 9001* de Ela Médical.

De façon caractéristique de l'invention, en cas d'une détection d'une FA installée, on modifie temporairement les paramètres de ces critères (paramètres nominaux ou paramètres programmés par le médecin), ou on ajoute un critère.

Par exemple, on allonge à 12, voire 16 cycles la durée de détection du paramètre de majorité, ou bien la durée de persistance (pour éviter d'allonger le temps de la thérapie, il est préférable de n'augmenter qu'un seul des ces deux paramètres, de préférence la durée de persistance).

On peut également porter le pourcentage de détection du paramètre de majorité à 80 %, voire 88 %, de préférence en augmentant simultanément le nombre de cycles de détection pour diminuer l'effet d'artefacts ou l'effet d'une sous-détection ventriculaire, par exemple en paramétrant le critère à 80 % sur 12 cycles ou à 88 % sur 16 cycles.

En variante ou en complément de cette modification temporaire des paramètres des critères, on peut modifier, également de façon temporaire, l'algorithme en introduisant un ou plusieurs critères supplémentaires, notamment pour confirmer qu'une TV ne s'est pas installée sur une FA.

Un premier critère supplémentaire, illustré sur les figures 1 à 3, consiste à adjoindre au critère de stabilité des intervalles RR un critère de recherche de cycles longs ou courts significativement éloignés de la zone de répartition moyenne des intervalles RR.

Cette zone de répartition moyenne, hachurée sur les figures, est définie comme la position du pic d'amplitude maximale dans l'histogramme RR (figure 1), ce pic étant borné par les intervalles RRmin et RRmax (la différence RRmax - RRmin étant définie par le paramétrage du critère de stabilité des intervalles RR, voir plus bas).

On définit ensuite de part et d'autre du pic deux zones Z1 et Z2, illustrées sur la figure 1 :
- une première zone Z1 située à proximité du pic, s'étendant par exemple de RRmin-StabRR à RRmin à gauche du pic, et de RRmax à RRmax+StabRR à droite du pic, et
- une seconde zone Z2 située à distance du pic, s'étendant par exemple de 0, ou de la valeur de la période réfractaire absolue ventriculaire, à RRmin-StabRR à gauche du pic, et de RRmax+StabRR à 600 ms à droite du pic.

On compare ensuite le nombre d'intervalles RR tombant dans Z2 (à distance du pic) à celui des intervalles RR tombant dans Z1 (proches du pic).

Si par exemple, comme illustré figure 2, au cours de huit cycles successifs on ne trouve aucun intervalle RR tombant dans Z2, on confirme la suspicion d'une TV nouvellement installée.

En revanche si, comme illustré figure 3, on trouve au moins une valeur d'intervalle RR tombant dans Z2, on confirme la suspicion d'une FA isolée et l'on poursuit l'exécution de l'algorithme sur cette base.

Un second critère supplémentaire qu'il est possible de prévoir consiste à comptabiliser le nombre d'événements auriculaires et d'événements ventriculaires sur une durée déterminée. Tant que le nombre d'événements auriculaires est supérieur au nombre d'événements ventriculaires, on confirme la suspicion d'une FA isolée. Si le nombre d'événements ventriculaires devient supérieur ou égal au nombre d'événements auriculaires, on confirme alors la suspicion d'une TV nouvellement installée (cas de "bitachycardie" FA + TV) et, dans ce cas, l'algorithme nominal de détection est rétabli avec ses paramètres nominaux.

Ces deux critères additionnels peuvent être utilisés séparément ou conjointement pour continuer à suspecter une FA isolée.

La modification, conformément à l'invention, des paramètres de l'algorithme et/ou l'introduction de critères additionnels peut être appliquée tant que le dispositif est en suspicion de TSV installée, que ce soit par l'algorithme de repli (tel que décrit dans le EP-A-0 488 840 au nom de ELA Médical) ou par tout autre moyen tel que capteur métabolique, capteur hémodynamique, etc. Dans les autres cas, les paramètres nominaux ou programmés par le médecin et l'algorithme nominal de détection sont appliqués.

Par ailleurs, si la modification des paramètres et/ou l'introduction de critères additionnels s'effectue plusieurs fois sur une période de 24 h, alors le dispositif appliquera ces modifications de façon permanente. Le nombre d'itérations avant que le dispositif n'applique ces modifications de façon permanente est de préférence égal à huit.

On peut également appliquer cette modification temporaire lorsqu'une TSV installée est confirmée par l'algorithme de détection des tachycardies (obtention d'une première persistance de TSV), les paramètres nominaux ou programmés par le médecin n'étant appliqués à nouveau que si le rythme se ralentit au-dessous de la fréquence de tachycardie programmée (typiquement 100 cpm).

## Revendications

1. Dispositif médical actif du type défibrillateur/cardioverteur implantable, comprenant :
- des moyens de délivrance d'impulsions à haute énergie,
- des moyens de recueil de l'activité ventriculaire et auriculaire,
- des moyens pour suspecter et confirmer la présence d'épisodes de tachycardie dans l'activité ainsi recueillie,
- des moyens classificateurs, propres à discriminer les tachycardies ainsi détectées entre tachycardies ventriculaires et tachycardies supra-ventriculaires en fonction de critères prédéterminés et d'un paramétrage donné de ces critères, et à autoriser le déclenchement des moyens de délivrance de la thérapie en présence de tachycardies ventriculaires et inhiber ces mêmes moyens en présence de tachycardies supra-ventriculaires,
dispositif **caractérisé en ce que** les moyens classificateurs comprennent des moyens propres à ajouter temporairement un ou plusieurs critères et/ou à modifier temporairement le paramétrage d'au moins l'un des critères existants en cas de tachycardie supra-ventriculaire.

2. Le dispositif médical de la revendication 1, dans lequel les critères ajoutés comprennent un critère supplémentaire de recherche d'intervalles RR courts et/ou longs significativement éloignés (Z2) d'une zone de stabilité (RRmax-RRmin) donnée.

3. Le dispositif médical de la revendication 2, dans lequel les zones d'éloignement significatif sont séparées des bornes (RRMin, RRmax) de la zone de stabilité par un intervalle (Z1) de largeur prédéterminée (StabRR), identique en-deçà et au-delà de la zone de stabilité.

4. Le dispositif médical de la revendication 1, dans lequel les critères ajoutés comprennent un critère supplémentaire de comptabilisation et de comparaison du nombre d'événements auriculaires par rapport à celui des événements ventriculaires pendant une durée donnée.

5. Le dispositif médical de la revendication 1, dans lequel le paramétrage modifiable comprend un paramètre de majorité dans lequel on évalue, sur un nombre de cycles donné, la présence d'un pourcentage, supérieur ou égal à un seuil donné, d'intervalles RR situés hors d'une zone de stabilité donnée (RRmax-RRmin).

6. Le dispositif médical de la revendication 5, dans lequel le paramétrage modifiable comprend un paramètre de durée de persistance des tachycardies.

7. Le dispositif médical de la revendication 5, dans lequel le paramétrage modifiable comprend un paramètre de largeur (RRmax-RRmin) d'une zone de stabilité des intervalles RR.

8. Le dispositif médical de la revendication 1, dans lequel les critères et/ou les paramètres des moyens classificateurs sont restaurés lorsque des épisodes de tachycardie supra-ventriculaire ne sont plus confirmés dans l'activité recueillie.

9. Le dispositif médical de la revendication 1, dans lequel, si un ou plusieurs critères sont ajoutés temporairement et/ou le paramétrage modifié temporairement un nombre déterminé de fois au cours d'une période donnée, cet ajout et/ou cette modification sont alors appliqués de façon permanente.

10. Le dispositif médical de la revendication 1, comprenant en outre des moyens pour délivrer une thérapie auriculaire en réponse à la détection d'une tachycardie supra-ventriculaire.

## Claims

1. An active medical device of the defibdllator/cardioverter type, comprising:
- means for delivering high energy pulses,
- means for detecting atrial and ventricular activity,
- means for suspecting and confirming the presence of tachycardia episodes in the detected activity,
- classification means, operative to discriminate among detected tachycardias between ventricular tachycardias and supra-ventricular tachycardias according to predetermined criteria and a given adjustment of these criteria, and to authorize triggering means for delivering a therapy in the presence of ventricular tachycardias, and to inhibit delivery of the therapy in the presence of a supra-ventricular tachycardia,
said device being **characterized in that** the classification means include means adapted to temporarily add one or several criteria and/or to temporarily modify the adjustment of at least one of the existing criteria in case of a supra-ventricular tachycardia.

2. The medical device of claim 1, wherein the added criteria comprise a supplementary criterion for research of RR short and/or long intervals significantly remote (Z2) from a given stability range (RRmax-RRmin).

3. The medical device of claim 1, wherein the significantly remote areas are separated from the limits (RRmax, RRmin) of the stability range by an interval (Z1) of a predetermined width (StabRR), identical beyond and within the stability range.

4. The medical device of claim 1, wherein the added criteria comprise a supplementary criterion for counting and comparing the number of atrial events with respect to the number of ventricular events during a given duration.

5. The medical device of claim 1, wherein the modifiable adjustment comprises a majority parameter in which one evaluates, over a given number of cycles, the presence of a percentage, greater than or equal to a given threshold, of RR intervals that are outside of a given stability zone (RRmax-RRmin).

6. The medical device of claim 5, wherein the modifiable adjustment comprises a parameter for the duration of the persistence of tachycardias.

7. The medical device of claim 5, wherein the modifiable adjustment comprises a parameter for the width (RRmax-RRmin) of a stability zone of the RR intervals.

8. The medical device of claim 1, wherein the criteria and/or the parameters of the classification means are restored when episodes of supra-ventricular tachycardia are no longer confirmed in the detected activity.

9. The medical device of claim 1, wherein, if one or several criteria are temporarily added and/or the adjustment is temporarily modified a determined number of times in the course of a given period, this addition and/or modification are then applied in a permanent manner.

10. The medical device of claim 1, further comprising means for delivering an atrial therapy in response to the detection of a supra-ventricular tachycardia.

## Patentansprüche

1. Aktive medizinische Vorrichtung von der Art eines implantierbaren Defibrillators/Kardioverters, aufweisend:
- Mittel zur Erteilung von Impulsen hoher Energie,
- Mittel zum Aufnehmen der ventrikulären und Herzvorhofaktivität,
- Mittel zum Vermuten und Bestätigen des Vorhandenseins von Episoden der Tachykardie in der so aufgenommenen Aktivität,
- Klassifikationsmittel, die geeignet sind, die so erkannten Tachykardien zwischen ventrikulären Tachykardien und supraventrikulären Tachykardien abhängig von vorbestimmten Kriterien und einer gegebenen Parametrierung dieser Kriterien zu unterscheiden, und bei Vorhandensein von ventrikulären Tachykardien die Auslösung der Mittel zur Erteilung der Therapie zu autorisieren und dieselben Mittel bei Vorhandensein von supraventrikulären Tachykardien zu hemmen,
wobei die Vorrichtung **dadurch gekennzeichnet ist, dass** die Klassifikationsmittel geeignete Mittel aufweisen zum temporären Hinzufügen eines oder mehrerer Kriterien und/oder die Parametrierung wenigstens eines der bestehenden Kriterien temporär zu modifizieren im Falle einer supraventrikulären Tachykardie.

2. Medizinische Vorrichtung nach Anspruch 1, in welcher die hinzugefügten Kriterien aufweisen ein zusätzliches Kriterium der Suche nach kurzen und/oder langen Intervallen RR, die signifikant von einer gegebenen Stabilitätszone (RRmax-RRmin) entfernt (Z2) sind.

3. Medizinische Vorrichtung nach Anspruch 2, in welcher die Zonen der signifikanten Entfernung von Stabilitätszone durch Grenzen (RRmin, RRmax) durch ein Intervall (Z1) von vorherbestimmter Ausdehnung (StabRR) entfernt sind, welche identisch diesseits und jenseits der Stabilitätszone sind.

4. Medizinische Vorrichtung nach Anspruch 1, in welcher die hinzugefügten Kriterien aufweisen ein zusätzliches Kriterium der Buchung und des Vergleichs der Anzahl der Herzvorhofereignisse im Vergleich zu der der ventrikulären Ereignisse während einer gegebenen Dauer.

5. Medizinische Vorrichtung nach Anspruch 1, in welcher die veränderbare Parametrierung einen Mehrheitsparameter aufweist, in welchem über eine gegebene Anzahl von Zyklen das Vorhandensein eines Prozentsatzes größer oder gleich einer gegebenen Schwell ausgewertet wird, von außerhalb einer gegebenen Stabilitätszone (RRmax-RRmin) gelegenen Intervallen RR.

6. Medizinische Vorrichtung nach Anspruch 5, in welcher die veränderbare Parametrierung aufweist einen Parameter der Dauer des Anhaltens der Tachykardien.

7. Medizinische Vorrichtung nach Anspruch 5, in welcher die veränderbare Parametrierung aufweist einen Parameter der Ausdehnung (RRmax-RRmin) einer Stabilitätszone der Intervalle RR.

8. Medizinische Vorrichtung nach Anspruch 1, in welcher die Kriterien und/oder die Parameter der Klassifikationsmittel wiederhergestellt werden, wenn die Episoden der supraventrikulären Tachykardie in der aufgenommenen Aktivität nicht mehr bestätigt werden.

9. Medizinische Vorrichtung nach Anspruch 1, in welcher, wenn im Verlauf einer gegebenen Periode eine bestimmte Anzahl von Malen ein oder mehrere Kriterien temporär hinzugefügt werden und/oder die Parametrierung temporär verändert wird, dann diese Hinzufügung und/oder diese Veränderung auf dauerhafte Art angewendet werden.

10. Medizinische Vorrichtung nach Anspruch 1, außerdem aufweisend Mittel zum Erteilen einer Herzvorhoftherapie in Antwort auf die Erkennung einer supraventrikulären Tachykardie.
